(19) Europäisches Patentamt
European Patent Office
Office européen des brevets

(11) **EP 0 846 462 B1**

(12) **FASCICULE DE BREVET EUROPEEN**

(45) Date de publication et mention
de la délivrance du brevet:
**29.12.1999 Bulletin 1999/52**

(51) Int. Cl.⁶: **A61K 7/50**, A61K 7/48

(21) Numéro de dépôt: **97402527.2**

(22) Date de dépôt: **24.10.1997**

(54) **Composition cosmétique sous la forme d'un gel transparent moussant**

Kosmetische Zusammensetzung in Form eines schäumenden transparenten Gels

Cosmetic composition in the form of a foaming transparent gel

(84) Etats contractants désignés:
**AT BE CH DE ES FR GB IT LI NL SE**

(30) Priorité: **07.11.1996 FR 9613601**

(43) Date de publication de la demande:
**10.06.1998 Bulletin 1998/24**

(73) Titulaire: **L'OREAL**
**75008 Paris (FR)**

(72) Inventeur: **Maurin, Véronique**
**75005 Paris (FR)**

(74) Mandataire:
**Andral, Christophe André Louis**
**L'OREAL-DPI**
**6 rue Bertrand Sincholle**
**92585 Clichy Cedex (FR)**

(56) Documents cités:
EP-A- 0 357 561          WO-A-91/07943
FR-A- 2 258 833          GB-A- 2 151 657

- DATABASE WPI Week 9612 Derwent
  Publications Ltd., London, GB; AN 96-112601
  XP002036849 "Clear gel for hair - consists of
  water-soluble polymer, polyhydric alcohol and
  foaming agent" & JP 08 012 533 A (KANEBO), 11
  janvier 1996
- DATABASE WPI Week 9602 Derwent
  Publications Ltd., London, GB; AN 96-017105
  XP002037113 "Make-up cleaner having high
  detergency - contg. polyoxyethylene monoester
  and maltitol ether" & JP 07 291 828 A (SHISEIDO)
  , 7 novembre 1995

**Description**

L'invention a pour objet une composition nettoyante sous la forme d'un gel aqueux transparent moussant à base d'acides alkyléther carboxyliques à chaîne grasse et de diols gras.

Le nettoyage de la peau est très important pour le soin du visage et du corps. Il doit être le plus performant possible car les résidus gras tels que l'excès de sébum, les restes des produits cosmétiques utilisés quotidiennement et les produits de maquillage, notamment les produits ⟨⟨waterproof⟩⟩ résistants à l'eau, s'accumulent dans les replis cutanés et à la surface de la peau et peuvent obstruer les pores de la peau, entraîner l'apparition de boutons. Une mauvaise qualité de nettoyage est souvent responsable, parmi d'autres facteurs de cause, d'un teint brouillé.

Traditionnellement, pour le nettoyage de la peau sont employées des compositions à base de sels alcalins d'acides gras (savons), souvent sous la forme de barres solides, ou à base de mélanges de ces savons avec des tensioactifs de synthèse. Si ces produits sont appréciés pour leurs propriétés nettoyantes, leur mousse et leur bonne rinçabilité, ils ne sont pas dépourvus d'inconvénients; en particulier, ils sont réputés décaper la peau, en détruisant le film hydro-lipidique de la peau et ils laissent la peau propre mais rêche.

Certains utilisateurs reprochent aux compositions nettoyantes exclusivement à base de tensioactifs de synthèse, sans savon, de laisser un film sur la peau après rinçage, propriété qui est souvent associée à une mauvaise rinçabilité. En outre, il est fréquemment reproché à ces compositions de dessécher la peau lorsqu'elles sont utilisées avec une trop grande fréquence. Enfin, ces compositions, si elles peuvent produire une mousse abondante, ne permettent pas d'obtenir une mousse ayant la texture fine, dense et serrée qui est caractéristique des savons.

En outre, lorsque l'on veut donner à une composition aqueuse tensio-active la consistance d'un gel, il est habituellement nécessaire de lui incorporer au moins un agent épaississant, comme par exemple un polymère épaississant ou des sels. L'incorporation de tels additifs dans les compositions tensio-actives aqueuses présente souvent l'inconvénient de nuire à la qualité de la mousse. En outre, les épaississants polymériques peuvent être coûteux.

On connaît, par exemple par le document WO90/0942, des compositions à base de tensioactifs anioniques et de diols gras. Ces compositions sont utilisées pour le nettoyage des textiles et utilisent comme tensioactifs anioniques des dérivés de la famille des alkylbenzène sulfonates et des alkyl sulfocarboxylates de méthyle.

On connaît également par le document FR-2258833, des produits pour les soins et la protection de la peau contenant des acides éther-carboxyliques polybasiques comme agents hydratants; par le document WO91/07943, des gels de rasage à moussage différé contenant le 2-éthyl-1,3-hexanediol ou le 2-méthylpentane-2,4-diol pour donner de la consistance aux gels et leur communiquer de la transparence; et par le document GB-2151657, dans son exemple 7, des gels-crèmes de rasage stables contenant, outre un savon d'acide gras, de l'acide trideceth-7 carboxylique.

Il subsiste donc le besoin d'un produit pour le nettoyage de la peau ayant les avantages d'un savon en terme de qualité de mousse et de rinçabilité, et n'ayant pas les inconvénients des savons (sécheresse, irritation), ce produit étant par ailleurs d'un aspect agréable et d'une consistance qui favorise une manipulation aisée.

L'invention a donc pour objet une composition cosmétique sous la forme d'un gel transparent moussant, comprenant au moins un acide alkyléther carboxylique à chaîne grasse, au moins un diol à chaîne grasse, et de l'eau, les quantités en poids d'acide alkyléther carboxylique à chaîne grasse [AK], et de diol à chaîne grasse [AG], par rapport au poids total de la composition vérifiant la relation : $[AG]/[AK] \leq 1/3,5$, et encore plus préférentiellement : $[AG]/[AK] \leq 1/4$.

Les compositions selon l'invention se caractérisent (1) par leur consistance, qui est celle d'un gel, (2) par leur transparence, qui leur confère un aspect attrayant et (3) par leur capacité à former une mousse abondante, fine, dense, serrée et onctueuse.

La texture de la mousse des compositions selon l'invention rappelle celle des savons.

En outre, elles possèdent une rinçabilité élevée et elles ne laissent pas de film sur la peau, propriétés qui les rendent comparables aux savons traditionnels.

En revanche, les compositions selon l'invention sont incomparablement plus douces et mieux tolérées que les savons. Elles laissent la peau nette mais non desséchée.

Enfin, ces compositions sont parfaitement stables au stockage.

Le mot ⟨⟨ transparence ⟩⟩ signifie qu'au travers d'une bouteille de verre transparent contenant la composition on peut distinguer les caractères imprimés sur une page de journal placée derrière cette bouteille.

L'acide alkyléther carboxylique à chaîne grasse est avantageusement choisi parmi les produits répondant à la formule (I) :

$$R\text{-}(O\text{-}C_nH_{2n})_p\text{-}O\text{-}A\text{-}COOM \tag{I}$$

dans laquelle :

- R représente une chaîne alkyle en $C_8$-$C_{30}$ linéaire ou ramifiée saturée ou insaturée,

- A représente un radical alcane di-yle en $C_1$-$C_6$, linéaire ou ramifié, saturé ou insaturé,
- M représente l'atome d'hydrogène, un métal alcalin ou alcalino-terreux, un cation ammonium ou un cation d'une base organique
- n = 2 ou 3,
- p est un entier allant de 1 à 40.

Préférentiellement, dans la formule (I) :

- R représente une chaîne alkyle en $C_{10}$-$C_{22}$,
- A représente un radical choisi parmi :-$CH_2$- ; -$CH_2$-$CH_2$- ; -$CH_2$-$CH_2$-$CH_2$-
- M représente l'atome d'hydrogène,
- n = 2, et/ou
- p est un entier allant de 2 à 30.

Encore plus préférentiellement :

- R représente une chaîne alkyle en $C_{12}$-$C_{18}$,
- A représente le radical -$CH_2$-
- M représente l'atome d'hydrogène,
- n = 2, et/ou
- p est un entier allant de 3 à 20.

De tels produits sont bien connus de l'homme du métier. Ils sont par exemple commercialisés par la société CHEM Y sous le nom de marque AKYPO RLM.

Le diol à chaîne grasse est choisi avantageusement parmi les alcools organiques comportant deux fonctions hydroxyle et une chaîne hydrocarbonée en $C_{10}$-$C_{22}$, préférentiellement en $C_{12}$-$C_{18}$.

De préférence on choisit un alcane diol à chaîne non ramifiée. De préférence on choisit un alcane diol à chaîne saturée. Avantageusement c'est un 1,2-alcane diol. On peut citer parmi les diols à chaîne grasse utilisables dans la présente invention : le 1,2-décane diol, le 1,2-dodécane diol, le 1,2-tétradécane diol, le 1,2-hexadécane diol, le 1,2-octadécane diol, ainsi que leurs mélanges.

De tels composés sont bien connus de l'homme du métier et sont disponibles commercialement, par exemple sous la marque LANETTE, auprès de la société HENKEL.

Les compositions selon l'invention ont la consistance d'un gel, sans qu'il soit besoin de leur incorporer d'additif épaississant. De préférence, ces compositions ont une viscosité Brookfield à 25°C supérieure ou égale à 1 Pa.s, et encore plus préférentiellement à 4 Pa.s.

Afin d'obtenir un gel d'une consistance satisfaisante et d'une limpidité parfaite, on choisit de préférence des quantités en poids d'acide alkyléther carboxylique à chaîne grasse [AK], et/ou de diol à chaîne grasse [AG], par rapport au poids total de la composition de telle sorte qu'elles vérifient la relation : [AG]/ AK] $\leq$ 1/3,5 et encore plus préférentiellement : [AG]/ [AK] $\leq$ 1/4 .

De préférence, pour obtenir une composition ayant la consistance d'un gel de texture parfaitement homogène on choisit : 1 $\leq$ [AK] $\leq$ 35,

préférentiellement : 2 $\leq$ [AK] $\leq$ 25,

et encore plus préférentiellement : 5 $\leq$ [AK] $\leq$ 20.

Pour obtenir un gel susceptible de produire une mousse se rapprochant par sa texture de celle des savons on choisit de préférence : 0,5 $\leq$ [AG] ;

et encore plus préférentiellement : 1,5 $\leq$ [AG].

On peut prévoir que la composition selon l'invention comprenne par ailleurs d'autres composants, comme par exemple :

- des tensioactifs moussants pour permettre la formation d'une mousse plus volumineuse, ces moussants étant de préférence choisis dans la classe des tensio-actifs amphotères ; on peut se reporter au document 《 Encyclopedia of Chemical Technology, KIRK-OTHMER》, volume 22, p.385-392, 3ème édition, 1979, WILEY, où sont donnés des exemples de tels tensioactifs. Préférentiellement, on choisit les tensioactifs amphotères parmi les alkylbétaïnes, les alkylsulfobétaïnes, les alkylamidopropylbétaïnes, les alkylcarboxyglycinates de métal alcalin ou alcalino-terreux, les imidazolines. Parmi ces familles de tensioactifs, on peut citer les exemples suivants : la diméthyl bétaïne, la coco bétaïne, la coco-amidopropylbétaïne, la coco-amidopropyl hydroxysultaïne, le di-sodium coco-amphodiacétate.
- des épaississants non-ioniques qui ne détruisent ni la transparence ni l'innocuité des compositions selon l'invention et ne lui confèrent pas de propriétés filmogènes ; ces épaississants peuvent être choisis parmi :

a- les $C_1$-$C_6$ alcanolamides d'acides $C_8$-$C_{22}$ alkyl éther carboxyliques ;

b- les produits d'addition de 10 à 300 moles d'oxyde d'éthylène ou d'oxyde de propylène sur des esters partiels de polyols ayant 2 à 16 atomes de carbone et d'acides gras ayant 12 à 22 atomes de carbone ; parmi cette famille de produits, on choisit préférentiellement ceux ayant un taux d'estérification supérieur ou égal à 2 ;

c- les alcools gras en $C_{12}$-$C_{22}$ polyoxyéthylénés et/ou polyoxypropylénés et/ou polyglycérolés, comportant de 20 à 500 résidus oxyde d'éthylène et/ou oxyde de propylène et/ou glycérol,

d- les esters gras en $C_{12}$-$C_{22}$ de polyoxyéthylène et/ou de polyoxypropylène et/ou de polyglycérol, et/ou

e- les polymères blocs de polyéthylène glycol et/ou de polypropylène glycol.

L'ensemble de ces produits sont disponibles commercialement :

- les $C_1$-$C_6$ alcanolamides d'acides $C_8$-$C_{22}$ alkyl éther carboxylique sont par exemple commercialisés par la société HENKEL sous la marque COMPERLAN, ou par la société CHEM Y sous le nom de marque AMINOL ;
- les produits d'addition d'oxyde d'éthylène ou d'oxyde de propylène sur des esters partiels de polyols et d'acides gras sont par exemple commercialisés par la société BASF sous la marque CREMOPHOR, ou par la société AMERCHOL sous la marque GLUCAMATE ;
- les alcools gras en $C_{12}$-$C_{22}$ polyoxyéthylénés et/ou polyoxypropylénés et/ou polyglycérolés sont par exemple commercialisés par la société ICI sous la marque BRIJ,
- les esters gras en $C_{12}$-$C_{22}$ de polyoxyéthylène et/ou de polyoxypropylène et/ou de polyglycérol sont par exemple commercialisés par la société ICI sous la marque MYRJ ,
- les polymères blocs de polyéthylène glycol et/ou de polypropylène glycol sont par exemple commercialisés par la société ICI sous la marque SYNPERONIC .

Les compositions selon l'invention peuvent en outre comprendre, sans voir se détériorer leurs qualités, notamment leur aspect gélifié et leur pouvoir moussant :

- des huiles cosmétiques, jusqu'à 10%, préférentiellement 3%, en poids par rapport au poids total de la composition; la nature de ces huiles n'est pas déterminante pour la mise en oeuvre de l'invention, elles peuvent être choisies parmi :

    . les huiles végétales, parmi lesquelles on peut citer l'huile d'amande douce, l'huile de noisette, l'huile de pépin de raisin, l'huile d'olive ;
    . les huiles minérales, par exemple l'huile de paraffine, l'huile de vaseline et les huiles d'hydrocarbures de façon générale ;

- des huiles oxyéthylénées, jusqu'à 20%, préférentiellement 10%, en poids par rapport au poids total de la composition ; par exemple, on peut citer parmi cette catégorie l'huile d'amande douce oxyéthylénée (20OE) commercialisée par la société CRODA sous la marque CROVOL A40 ; l'huile d'olive oxyéthylénée (10OE) commercialisée par la société COSMETOCHEM sous le nom de : Huile d'olive W ; les triglycérides oxyéthylénés ;
- des agents colorants, nacrants, des pigments ; parmi les nacrants on peut citer par exemple les acides gras, les esters et les di-esters de glycol, les alcools gras ; parmi les pigments : les dérivés de la guanine ;
- des antioxydants, comme par exemple des sulfites ;
- des conservateurs ;
- des parfums ;
- des filtres ;
- des actifs hydrophiles ou lipophiles : les actifs pour la peau peuvent être des actifs anti-âge, des actifs anti-rides, des hydratants ou des humectants, des actifs dépigmentants, des actifs anti-radicaux libres (espèces radicalaires de l'oxygène), des actifs nutritifs, des actifs protecteurs, des actifs restructurants, des actifs raffermissants, des actifs amincissants, des actifs antiacnéiques, des actifs exfoliants, des actifs émollients ou encore des actifs traitants des maladies de peau comme les mycoses, les dermites, le psoriasis, etc. ; ces actifs sont utilisés, selon leur nature, dans les proportions habituelles et par exemple de 0,01 % à 10% en poids par rapport au poids total de la composition ; comme actifs anti-acnéiques, anti-âge, anti-ride, hydratants, exfoliants, on peut citer plus particulièrement les $\alpha$-hydroxy-acides (acides glycolique, lactique, malique, citrique, etc.).
- des charges insolubles : talc, kaolin, poudre de polyéthylène, particules de polyamide comme par exemple celles vendues sous la dénomination "ORGASOL" par la Société ATOCHEM également connues sous les noms (CTFA) de "polyamide 12" ou "polyamide 6" ; on peut également utiliser dans ces compositions des poudres de Nylon répertoriées sous le nom CTFA de "Nylon 12" ou "Nylon 6"; de telles compositions sont intéressantes dans le nettoyage de peau pour leurs propriétés exfoliantes.

La composition selon l'invention comprend en outre de l'eau. Habituellement, on entend par eau de l'eau pure. Toutefois, tout ou partie de l'eau utilisée dans les compositions selon l'invention peut éventuellement être choisie parmi les eaux minérales et/ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Uriage-les-bains, l'eau d'Avene.

De préférence les compositions selon l'invention ont un pH allant de 4,5 à 7,5, afin de leur garantir une tolérance parfaite par la peau.

Les compositions selon l'invention peuvent se présenter sous la forme d'une composition de nettoyage pour la peau et/ou les cheveux, d'une composition exfoliante, d'une composition démaquillante, d'une composition de gommage, d'une composition hydratante.

Un autre objet de la présente invention est l'utilisation des compositions selon l'invention telles que ci-dessus définies comme, ou pour la fabrication de, compositions cosmétiques et/ou dermatologiques pour application sur la peau ou les cheveux, en particulier pour le nettoyage et/ou le soin de la peau.

Les compositions selon l'invention, particulièrement douces, sont tout à fait adaptées au nettoyage et/ou au soin des peaux sensibles.

## EXEMPLES

Dans les exemples les noms des produits sont les noms INCI. Pour chaque composition, les pourcentages sont donnés en poids de matière active par rapport au poids total de la composition. La viscosité est mesurée à 25°C à l'aide d'un viscosimètre Brookfield dont la vitesse du module tournant (module I) est fixée à 5 tours/min.

Afin d'illustrer les avantages des compositions selon l'invention, on a préparé des essais comparatifs :

Essai I (Gel selon l'invention) :

| Laureth-11 carboxylic acid | 20 | % |
| Lauryl glycol | 5 | % |
| Cocoamidopropyl hydroxysultaine | 3,5 | % |
| PEG-160 sorbitane triiso-stéarate | 2 | % |
| PEG-7 glycéryl cocoate | 2 | % |
| Glycérine | 1 | % |
| Conservateur | qs | |
| Eau déminéralisée | qsp 100% | |

Mode opératoire et caractéristiques : après mélange homogène de tous les ingrédients, on obtient un gel transparent, limpide, incolore ayant une viscosité de 6 Pa.s et un pH égal à 5. Le rapport [AG]/[AK] est égal à 1/4.

Essai II (produit selon l'art antérieur) :

| Sodium Laureth Sulfate | 20 | % |
|---|---|---|
| Lauryl glycol | 5 | % |
| Cocoamidopropyl hydroxysultaine | 3,5 | % |
| PEG-160 sorbitane triiso-stéarate | 2 | % |
| PEG-7 glycéryl cocoate | 2 | % |
| Glycérine | 1 | % |
| Conservateur | qs | |
| Eau déminéralisée | qsp 100% | |

Mode opératoire et caractéristiques : après mélange homogène de tous les ingrédients, on obtient un liquide trouble, ayant une viscosité de 0,36 Pa.s. Cette composition est instable et se décompose en deux phases après 24h de stockage à température ambiante.

On a fait tester les compositions des essais I et II à un panel de 18 personnes en leur demandant de déterminer laquelle des compositions répondait le mieux aux caractéristiques de ⟨⟨mousse abondante⟩⟩, ⟨⟨mousse fine⟩⟩, ⟨⟨bonne qualité de nettoyage⟩⟩ et d'attribuer une note allant de 1 à 5 sur la satisfaction de chacun de ces critères (5=critère satisfait ; 1=critère non satisfait).

Résultats :

- Nombre de personnes ayant élu chaque essai :

| | Essai I | Essai II |
|---|---|---|
| mousse abondante : | 11 | 7 |
| mousse fine : | 8 | 4 |

- Pour le critère de bonne qualité de nettoyage, le total des points attribués à chaque essais est sensiblement équivalent.

On constate donc que les compositions selon l'invention permettent d'obtenir une qualité de nettoyage équivalente à une composition détergente selon les enseignements de l'art antérieur. En revanche les compositions selon l'invention permettent d'obtenir une mousse plus abondante et plus fine que les compositions selon l'art antérieur. En outre, ces compositions sont d'une parfaite innocuité, en particulier elles ne dessèchent pas et n'irritent pas la peau et elles ne déposent pas de film sur la peau.

Afin d'illustrer l'invention, on a préparé plusieurs compositions selon l'invention ainsi que des exemples comparatifs qui soulignent la pertinence des critères exposés ci-dessus définissant l'invention. Ces exemples sont décrits dans le tableau 1 ci-dessous :

- exemples selon l'invention : 1, 2, 3, 7, 8, 9, 10
- exemples en dehors de l'invention : 4, 5, 6

| EXEMPLES | 1 | 2 | 3 | 4 | 5 | 6 | 7 | 8 | 9 | 10 |
|---|---|---|---|---|---|---|---|---|---|---|
| Laureth-11 carboxylic acid | 25 | - | 7,5 | 7,5 | 7,5 | 7,5 | 20 | 20 | 10 | 20 |
| Laureth-5 carboxylic acid | - | 13,5 | 6,75 | 6,75 | 6,5 | 6,75 | - | - | - | - |
| Lauryl glycol | 5 | 3 | 3 | 6 | 4,6 | - | 5 | 5 | 2,5 | 5 |
| Cocoamidopropyl hydroxy-sultaine | 3,5 | 3,5 | 3,5 | 6 | 6 | 3,5 | 5 | 5 | 2,5 | - |
| PEG-160 sorbitane triiso-stéarate | 2 | 2 | 2 | 2 | 2 | 2 | 2 | 2 | 1 | 2 |
| Eau déminéralisée | qsp 100 | qsp 100 | qsp100 | qsp 100 | qsp 100 | qsp 100 | qsp 100 | qsp 100 | qsp 100 | qsp 100 |
| Stéarylalcohol | - | - | - | - | - | 3 | - | - | - | - |
| Glycol distéarate (agent na-crant) | - | - | - | - | - | - | - | 2 | - | - |
| Résultat pH | 5,5 | 5,7 | 5,1 | 7 | 7 | 7 | 5 | 5,6 | 7,1 | 5 |
| Viscosité (Pa.s) | 22,7 | 11,2 | 7,1 | 8,5 | - | - | 6,0 | 6,2 | 1,9 | 10 |
| [AG]/[AK] | 1/5 | 1/4,5 | 1/4,7 | 1/2,4 | 1/3 | | 1/4 | 1/4 | 1/4 | 1/4 |
| OBSERVATIONS | gel limpide | gel limpide | gel limpide | gel opaque instable | gel opaque instable | liquide aspect laiteux instable | gel limpide | gel nacré | gel limpide | gel limpide |

TABLEAU 1

EP 0 846 462 B1

**Revendications**

1. Composition cosmétique sous la forme de, ou comprenant, un gel transparent moussant caractérisée en ce qu'elle comprend :

   (i) au moins un acide alkyléther carboxylique à chaîne grasse,
   (ii) au moins un diol à chaîne grasse, et
   (iii) de l'eau,

   les quantités en poids d'acide alkyléther carboxylique à chaîne grasse [AK], et de diol à chaîne grasse [AG], par rapport au poids total de la composition vérifiant la relation : $[AG]/[AK] \leq 1/3,5$ .

2. Composition selon la revendication précédente, caractérisée en ce qu'elle a une viscosité Brookfield à 25°C supérieure ou égale à 1 Pa.s, et préférentiellement à 4 Pa.s.

3. Composition selon l'une quelconque des revendications précédentes, caractérisée en ce que l'acide alkyléther carboxylique à chaîne grasse est choisi parmi les produits répondant à la formule (I) :

$$R-(O-C_nH_{2n})_p-O-A-COOM \qquad\qquad (I)$$

   dans laquelle :

   - R représente une chaîne alkyle en $C_8$-$C_{30}$ linéaire ou ramifiée saturée ou insaturée,
   - A représente un radical alcane di-yle en $C_1$-$C_6$, linéaire ou ramifié, saturé ou insaturé,
   - M représente l'atome d'hydrogène, un métal alcalin ou alcalino-terreux, un cation ammonium ou un cation d'une base organique,
   - n = 2 ou 3,
   - p est un entier allant de 1 à 40.

4. Composition selon la revendication précédente, caractérisée en ce que l'acide alkyléther carboxylique à chaîne grasse est choisi parmi les produits répondant à la formule (I) dans laquelle :

   - R représente une chaîne alkyle en C10-C22,
   - A représente un radical choisi parmi :-$CH_2$- ; -$CH_2$-$CH_2$- ; -$CH_2$-$CH_2$-$CH_2$-
   - M représente l'atome d'hydrogène
   - n = 2, et/ou
   - p est un entier allant de 2 à 30.

5. Composition selon la revendication précédente, caractérisée en ce que l'acide alkyléther carboxylique à chaîne grasse est choisi parmi les produits répondant à la formule (I) dans laquelle :

   - R représente une chaîne alkyle en C12-C18,
   - A représente le radical -$CH_2$-
   - M représente l'atome d'hydrogène,
   - n = 2, et/ou
   - p est un entier allant de 3 à 20.

6. Composition selon l'une quelconque des revendications précédentes, caractérisée en ce que le diol à chaîne grasse est choisi parmi les alcools organiques comportant deux fonctions hydroxyle et une chaîne hydrocarbonée en $C_{10}$-$C_{22}$, préférentiellement en $C_{12}$-$C_{18}$.

7. Composition selon la revendication 6, caractérisée en ce que le diol à chaîne grasse est choisi parmi : le 1,2-décane diol, le 1,2-dodécane diol, le 1,2-tétradécane diol, le 1,2-hexadécane diol, le 1,2-octadécane diol, leurs mélanges.

8. Composition selon la revendication 1, caractérisée en ce que : $[AG]/[AK] \leq 1/4$ .

9. Composition selon l'une quelconque des revendications 1 et 8, caractérisée en ce que: $1 \leq [AK] \leq 35$.

**10.** Composition selon la revendication 9, caractérisée en ce que : $2 \leq [AK] \leq 25$.

**11.** Composition selon l'une quelconque des revendications 1 à 10, caractérisée en ce que: $0,5 \leq [AG]$.

**12.** Composition selon l'une quelconque des revendications précédentes, caractérisée en ce qu'elle comprend par ailleurs au moins un autre composant choisi parmi :

- les tensioactifs moussants, préférentiellement, des tensioactifs amphotères ;
- les épaississants non-ioniques qui peuvent être choisis parmi :

  a- les $C_1$-$C_6$ alcanolamides d'acides C8-C22 alkyl éther carboxyliques ;
  b- les produits d'addition de 10 à 300 moles d'oxyde d'éthylène ou d'oxyde de propylène sur des esters parties de polyols ayant 2 à 16 atomes de carbone et d'acides gras ayant 12 à 22 atomes de carbone ; parmi cette famille de produits, on choisit préférentiellement ceux ayant un taux d'estérification supérieur ou égal à 2 ;
  c- les alcools gras en $C_{12}$-$C_{22}$ polyoxyéthylénés et/ou polyoxypropylénés et/ou polyglycérolés, comportant de 20 à 500 résidus oxyde d'éthylène et/ou oxyde de propylène et/ou glycérol,
  d- les esters gras en $C_{12}$-$C_{22}$ de polyoxyéthylène et/ou de polyoxypropylène et/ou de polyglycérol, et/ou
  e- les polymères blocs de polyéthylène glycol et/ou de polypropylène glycol.

- les huiles cosmétiques ;
- les huiles oxyéthylénées ;
- les agents colorants, nacrants, des pigments ;
- les antioxydants, comme par exemple des sulfites ;
- les conservateurs ;
- les parfums ;
- les filtres ;
- les actifs hydrophiles ou lipophiles ;
- les charges insolubles.

**13.** Composition selon l'une quelconque des revendications précédentes, caractérisée en ce qu'elle comprend au moins une eau thermale ou minérale naturelle choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Uriage-les-bains, l'eau d'Avene.

**14.** Composition selon l'une quelconque des revendications précédentes, caractérisée en ce qu'elle a un pH allant de 4,5 à 7,5.

**15.** Composition selon l'une quelconque des revendications précédentes, caractérisée en ce qu'elle se présente la forme d'une composition de nettoyage pour la peau et/ou les cheveux, d'une composition exfoliante, d'une composition démaquillante, d'une composition de gommage, d'une composition hydratante.

**16.** Utilisation d'une composition selon l'une quelconque des revendications précédentes pour la fabrication de compositions cosmétiques et/ou dermatologiques pour application sur la peau ou les cheveux, en particulier pour le nettoyage et/ou le soin de la peau.

**17.** Utilisation selon la revendication 16, pour le nettoyage et/ou le soin des peaux sensibles.

**Claims**

**1.** Cosmetic composition in the form of, or comprising, a transparent foaming gel, characterized in that it comprises:

  (i) at least one alkyl ether carboxylic acid with a fatty chain,
  (ii) at least one diol with a fatty chain, and
  (iii) water,

the amounts by weight of alkyl ether carboxylic acid with a fatty chain [AK] and of diol with a fatty chain [AG], with respect to the total weight of the composition, obeying the relationship: [AG]/[AK] ≤ 1/3.5 .

2. Composition according to the preceding claim, characterized in that it has a Brookfield viscosity at 25°C of greater than or equal to 1 Pa • s and preferably greater than or equal to 4 Pa • s.

3. Composition according to either one of the preceding claims, characterized in that the alkyl ether carboxylic acid with a fatty chain is chosen from the products corresponding to the formula (I):

$$R\text{-}(O\text{-}C_nH_{2n})_p\text{-}O\text{-}A\text{-}COOM \tag{I}$$

in which:

- R represents a saturated or unsaturated, linear or branched $C_8$-$C_{30}$ alkyl chain,
- A represents a saturated or unsaturated, linear or branched $C_1$-$C_6$ alkanediyl radical,
- M represents the hydrogen atom, an alkali metal or alkaline-earth metal, an ammonium cation or a cation of an organic base,
- n = 2 or 3,
- p is an integer ranging from 1 to 40.

4. Composition according to the preceding claim, characterized in that the alkyl ether carboxylic acid with a fatty chain is chosen from the products corresponding to the formula (I) in which:

- R represents a $C_{10}$-$C_{22}$ alkyl chain,
- A represents a radical chosen from: $-CH_2-$, $-CH_2-CH_2-$ or $-CH_2-CH_2-CH_2-$,
- M represents the hydrogen atom,
- n = 2, and/or
- p is an integer ranging from 2 to 30.

5. Composition according to the preceding claim, characterized in that the alkyl ether carboxylic acid with a fatty chain is chosen from the products corresponding to the formula (I) in which:

- R represents a $C_{12}$-$C_{18}$ alkyl chain,
- A represents the $-CH_2-$ radical,
- M represents the hydrogen atom,
- n = 2, and/or
- p is an integer ranging from 3 to 20.

6. Composition according to any one of the preceding claims, characterized in that the diol with a fatty chain is chosen from organic alcohols containing two hydroxyl functional groups and a $C_{10}$-$C_{22}$, preferably $C_{12}$-$C_{18}$, hydrocarbon chain.

7. Composition according to Claim 6, characterized in that the diol with a fatty chain is chosen from: 1,2-decanediol, 1,2-dodecanediol, 1,2-tetradecanediol, 1,2-hexadecanediol, 1,2-octadecanediol and their mixtures.

8. Composition according to Claim 1, characterized in that: [AG]/[AK] ≤ 1/4 .

9. Composition according to either one of Claims 1 and 8, characterized in that: 1 ≤ [AK] ≤ 35.

10. Composition according to Claim 9, characterized in that: 2 ≤ [AK] ≤ 25.

11. Composition according to any one of Claims 1 to 10, characterized in that: 0.5 ≤ [AG].

12. Composition according to any one of the preceding claims, characterized in that it furthermore comprises at least one other component chosen from:

- foaming surfactants, preferably amphoteric surfactants;
- non-ionic thickeners which can be chosen from:

a- $C_1$-$C_6$ alkanolamides of $C_8$-$C_{22}$ alkyl ether carboxylic acids;

b- addition products of 10 to 300 mol of ethylene oxide or of propylene oxide with partial esters of polyols having 2 to 16 carbon atoms and of fatty acids having 12 to 22 carbon atoms; among this family of products, it is preferable to choose those having a degree of esterification of greater than or equal to 2;

c- polyoxyethylenated and/or polyoxypropylenated and/or polyglycerolated $C_{12}$-$C_{22}$ fatty alcohols containing from 20 to 500 ethylene oxide and/or propylene oxide and/or glycerol residues,

d- $C_{12}$-$C_{22}$ fatty esters of polyoxyethylene and/or of polyoxypropylene and/or of polyglycerol, and/or

e- block polymers of polyethylene glycol and/or of polypropylene glycol;

- cosmetic oils;
- oxyethylenated oils;
- colouring agents, pearlescent agents or pigments;
- antioxidizing agents, such as, for example, sulphites;
- preservatives;
- fragrances;
- screening agents;
- hydrophilic or lipophilic active agents;
- insoluble fillers.

13. Composition according to any one of the preceding claims, characterized in that it comprises at least one natural thermal or mineral water chosen from water from Vittel, waters from the Vichy basin, water from Uriage, water from La Roche Posay, water from La Bourboule, water from Enghien-les-Bains, water from Saint Gervais-les-Bains, water from Néris-les-Bains, water from Allevard-les-Bains, water from Digne, water from Maizières, water from Neyrac-les-Bains, water from Lons-le-Saunier, water from Eaux-Bonnes, water from Rochefort, water from Saint Christau, water from Les Fumades, water from Tercis-les-Bains, water from Uriage-les-Bains and water from Avene.

14. Composition according to any one of the preceding claims, characterized in that it has a pH ranging from 4.5 to 7.5.

15. Composition according to any one of the preceding claims, characterized in that it is provided in the form of a cleaning composition for the skin and/or the hair, of an exfoliative composition, of a make-up removal composition, of a scrubbing composition or of a moisturizing composition.

16. Use of a composition according to any one of the preceding claims for the manufacture of cosmetic and/or dermatological compositions for application to the skin or the hair, in particular for cleaning and/or caring for the skin.

17. Use according to Claim 16, for cleaning and/or caring for sensitive skin.

**Patentansprüche**

1. Kosmetische Zusammensetzung, die in Form eines schäumenden, transparenten Gels vorliegt oder ein schäumendes, transparentes Gel enthält, dadurch gekennzeichnet, daß sie enthält:

    (i) mindestens eine Alkylethercarbonsäure mit Fettkette,
    (ii) mindestens ein Diol mit Fettkette, und
    (iii) Wasser,

    wobei die gewichtsbezogenen Mengenanteile der Alkylethercarbonsäure mit Fettkette [AK] und des Diols mit Fettkette [AG], bezogen auf das Gesamtgewicht der Zusammensetzung, die folgende Beziehung erfüllen: [AG]/[AK] $\leq$ 1/3,5 .

2. Zusammensetzung nach dem Vorhergehenden Anspruch, dadurch gekennzeichnet, daß sie eine Brookfield-Viskosität bei 25 °C von mindestens 1 Pa $\cdot$ s und vorzugsweise 4 Pa $\cdot$ s aufweist.

3. Zusammensetzung nach einem der vorhergehenden Ansprüche, dadurch gekennzeichnet, daß die AlkylethercarbonSäure mit Fettkette unter den Produkten der Formel (I) ausgewählt ist

$$R\text{-}(O\text{-}C_nH_{2n})_p\text{-}O\text{-}A\text{-}COOM \qquad (I),$$

worin bedeuten:

- R eine geradkettige oder verzweigte, gesättigte oder ungesättigte $C_{8-30}$-Alkylgruppe,
- A eine geradkettige oder verzweigte, gesättigte oder ungesättigte $C_{1-6}$-Alkandiylgruppe,
- M Wasserstoff, ein Alkali- oder Erdalkalimetall, ein Ammoniumkation oder ein Kation einer organischen Base,
- n = 2 oder 3, und
- p eine ganze Zahl im Bereich von 1 bis 40.

4. Zusammensetzung nach dem vorhergehenden Anspruch, dadurch gekennzeichnet, daß die Alkylethercarbonsäure mit Fettkette unter den Produkten der Formel (I) ausgewählt ist, worin bedeuten:

- R eine $C_{10-22}$-Alkylgruppe,
- A eine Gruppe, die ausgewählt ist unter: $-CH_2-$; $-CH_2-CH_2-$; $-CH_2-CH_2-CH_2-$,
- M ein Wasserstoffatom,
- n = 2, und/oder
- p eine ganze Zahl im Bereich von 2 bis 30.

5. Zusammensetzung nach dem vorhergehenden Anspruch, dadurch gekennzeichnet, daß die Alkylethercarbonsäure mit Fettkette unter den Produkten der Formel (I) ausgewählt ist, worin bedeuten:

- R eine $C_{12-18}$-Alkylgruppe,
- A die Gruppe $-CH_2-$,
- M ein Wasserstoffatom,
- n = 2, und/oder
- p eine ganze Zahl im Bereich von 3 bis 20.

6. Zusammensetzung nach einem der vorhergehenden Ansprüche, dadurch gekennzeichnet, daß das Diol mit Fettkette unter den organischen Alkoholen ausgewählt ist, die zwei Hydroxygruppen und eine Kohlenwasserstoffkette mit 10 bis 22 und vorzugsweise 12 bis 18 Kohlenstoffatomen aufweisen.

7. Zusammensetzung nach Anspruch 6, dadurch gekennzeichnet, daß das Diol mit Fettkette ausgewählt ist unter: 1,2-Decandiol, 1,2-Dodecandiol, 1,2-Tetradecandiol, 1,2-Hexadecandiol, 1,2-Octadecandiol und deren Gemischen.

8. Zusammensetzung nach Anspruch 1, dadurch gekennzeichnet, daß: $[AG]/[AK] \leq 1/4$ .

9. Zusammensetzung nach einem der Ansprüche 1 bis 8, dadurch gekennzeichnet, daß: $1 \leq [AK] \leq 35$.

10. Zusammensetzung nach Anspruch 9, dadurch gekennzeichnet, daß: $2 \leq [AK] \leq 25$.

11. Zusammensetzung nach einem der Ansprüche 1 bis 10, dadurch gekennzeichnet, daß: $0{,}5 \leq [AG]$.

12. Zusammensetzung nach einem der vorhergehenden Ansprüche, dadurch gekennzeichnet, daß sie ferner mindestens einen weiteren Bestandteil enthält, der ausgewählt ist unter:

- schäumenden grenzflächenaktiven Stoffen und vorzugsweise amphoteren grenzflächenaktiven Stoffen;
- nichtionischen Verdickungsmitteln, die ausgewählt sein können unter:

  (a) den $C_{1-6}$-Alkanolamiden von $C_{8-22}$-Alkylethercarbonsäuren;
  (b) den Additionsprodukten von 10 bis 300 mol Ethylenoxid oder Propylenoxid mit partiellen Estern von Polyolen mit 2 bis 16 Kohlenstoffatomen und Fettsäuren mit 12 bis 22 Kohlenstoffatomen; wobei aus dieser Produktgruppe vorzugsweise die Produkte mit einem Veresterungsgrad von mindestens 2 ausgewählt werden;
  (c) den polyethoxylierten und/oder polypropoxylierten und/oder mehrfach mit Glycerin veretherten $C_{12-22}$-Fettalkoholen, die 20 bis 500 Ethylenoxid- und/oder Propylenoxid- und/oder Glycerineinheiten aufweisen;
  (d) den $C_{12-22}$-Fettsäureestern von Polyethylenoxid und/oder Polypropylenoxid und/oder Polyglycerin und/oder
  (e) Blockpolymeren von Polyethylenglykol und/oder Polypropylenglykol;

- kosmetischen Ölen;
- ethoxylierten Ölen;
- Färbemitteln, Perglanzmitteln, Pigmenten;
- Antioxidantien, wie beispielsweise Sulfiten;
- Konservierungsmitteln;
- Parfums;
- Filtern;
- hydrophilen oder lipophilen Wirkstoffen; und
- unlöslichen Füllstoffen.

13. Zusammensetzung nach einem der vorhergehenden Ansprüche, dadurch gekennzeichnet, daß sie mindestens ein natürliches Thermalwasser oder Mineralwasser enthält, das ausgewählt ist unter:
Wasser aus Vittel, Wasser aus dem Vichybecken, Wasser aus Uriage, Wasser aus Roche Posay, Wasser aus Bourboule, Wasser aus Enghien-Les-Bains, Wasser aus Saint Gervais-les-Bains, Wasser aus Néris-les-Bains, Wasser aus Allevard-les-Bains, Wasser aus Digne, Wasser aus Maizières, Wasser aus Neyrac-les-Bains, Wasser aus Lonsle-Saunier, Eaux-Bonnes, Wasser aus Rochefort, Wasser aus Saint Christau, Wasser aus Fumades, Wasser aus Tercis-les-Bains, Wasser aus Uriage-les-Bains und Wasser aus Avene.

14. Zusammensetzung nach einem der vorhergehenden Ansprüche, dadurch gekennzeichnet, daß sie einen pH-Wert im Bereich von 4,5 bis 7,5 aufweist.

15. Zusammensetzung nach einem der vorhergehenden Ansprüche, dadurch gekennzeichnet, daß sie in Form einer Zusammensetzung zur Reinigung der Haut und/oder der Haare, einer Zusammensetzung zur Exfoliation, einer Zusammensetzung zum Abschminken, einer Peeling-Zusammensetzung oder einer hydratisierenden Zusammensetzung vorliegt.

16. Verwendung einer Zusammensetzung nach einem der vorhergehenden Ansprüche zur Herstellung von kosmetischen und/oder dermatologischen Zusammensetzungen zum Auftragen auf die Haut oder die Haare und insbesondere zur Reinigung und/oder zur Pflege der Haut.

17. Verwendung nach Anspruch 16 zur Reinigung und/oder zur Pflege von empfindlicher Haut.